# EUROPÄISCHE PATENTSCHRIFT

(11) **EP 2 744 914 B1**
(45) Veröffentlichungstag und Bekanntmachung des Hinweises auf die Patenterteilung: **27.04.2016**
(21) Anmeldenummer: 12766597.4
(22) Anmeldetag: 15.08.2012
(51) Int. Cl.: C07D 239/47, C12Q 1/68

(54) **VERWENDUNG VON MAD2L2 ALS STRATIFIKATIONSMARKER BEI DER BEHANDLUNG VON BRUSTTUMOREN MIT NEUEN PAN-CDK-INHIBITOREN**
USE OF MAD2L2 AS A STRATIFICATION MARKER IN THE TREATMENT OF BREAST TUMOURS WITH NOVEL PAN-CDK INHIBITORS
UTILISATION DE MAD2L2 COMME MARQUEUR DE STRATIFICATION DANS LE TRAITEMENT DE TUMEURS DU SEIN AVEC DE NOUVEAUX INHIBITEURS DE KINASES CYCLINE-DÉPENDANTES (CDK) PAN

(30) Priorität: 16.08.2011 DE 102011080992
(43) Veröffentlichungstag der Anmeldung: 25.06.2014
(73) Patentinhaber: Bayer Intellectual Property GmbH, 40789 Monheim (DE)
(72) Erfinder: SIEMEISTER, Gerhard, 13503 Berlin (DE); GROTH, Philip, 10717 Berlin (DE)
(74) Vertreter: BIP Patents
(86) Internationale Anmeldenummer: PCT/EP2012/065944
(87) Internationale Veröffentlichungsnummer: WO 2013/024116

(56) Entgegenhaltungen:
- EP-A1- 2 179 991
- RIMKUS C ET AL.: "Expression of the mitotic checkpoint gene MAD2L2 has prognostic significance in colon cancer", INTERNATIONAL JOURNAL OF CANCER, Bd. 120, 2006, Seiten 207-211, XP002687928, in der Anmeldung erwähnt
- HUNT K K ET AL: "Cyclin E as a prognostic and predictive marker in breast cancer", SEMINARS IN CANCER BIOLOGY, SAUNDERS SCIENTIFIC PUBLICATIONS, PHILADELPHIA, PA, US, Bd. 15, Nr. 4, 1. August 2005 (2005-08-01) , Seiten 319-326, XP004995903, ISSN: 1044-579X, DOI: 10.1016/J.SEMCANCER.2005.04.007 in der Anmeldung erwähnt

## Beschreibung

Die Erfindung betrifft die Verwendung von MAD2L2 als Stratifikationsmarker bei der Behandlung von Brusttumoren mit neuen pan-CDK-Inhibitoren

Der eukaryote Zellteilungszyklus stellt die Duplikation des Genoms und seine Verteilung auf die Tochterzellen sicher, indem er eine koordinierte und regulierte Abfolge von Ereignissen durchläuft. Der Zellzyklus wird in vier aufeinanderfolgende Phasen eingeteilt: die G1 Phase repräsentiert die Zeit vor der DNA-Replikation in der die Zelle wächst. In der S Phase repliziert die Zelle ihre DNA, und in der G2 Phase bereitet sie sich auf den Eintritt in die Mitose vor. In der Mitose (M Phase) wird die replizierte DNA getrennt und die Zellteilung vollzogen. Die Zyklin-abhängigen Kinasen (CDKs), eine Familie von Serin/Threonin-Kinasen, deren Mitglieder die Bindung eines Zyklins (Cyc) als regulatorische Untereinheit zu ihrer Aktivierung benötigen, treiben die Zelle durch den Zellzyklus. Unterschiedliche CDK/Cyc Paare sind in den verschiedenen Phasen des Zellzyklus aktiv. Für die grundlegende Funktion des Zellzyklus bedeutende CDK/Cyc Paare sind beispielsweise CDK4(6)/CycD, CDK2/CycE, CDK2/CycA, CDK1/CycA und CDK1/CycB. So treiben zum Beispiel die Aktivitäten der CDK4(6)/CycD und CDK2/CycE Komplexe den Eintritt einer Zelle in den Zellzyklus an und das Durchlaufen des "Restriction Point", der die Unabhängigkeit einer Zelle von weiteren Wachstumssignalen für den Abschluss der begonnenen Zellteilung markiert.

Eine Reihe an Kontrollmechanismen stellen den geordneten Ablauf der Zellteilungsphasen und die korrekte Verteilung des duplizierten genetischen Materials auf die Tochterzellen sicher. Unter anderem wird die Aktivität der CDKs durch inhibitorische Proteine, wie zum Beispiel p21, p16, oder p27, beeinflußt, und die Expression und der Abbau der Cycline reguliert. Die Proteine des Spindle-Assembly Checkpoint stellen während der Mitosephase des Zellteilungszyklus die korrekte Anheftung des Spindelapparates an die duplizierten Chromosomen sicher und sorgen für eine korrekte Verteilung der Chormosomen auf die Tochterzellen. Wesentliche Proteine des Spindle-Assembly Checkpoints sind MAD1, MAD2, BUB1, BUBR1, TTK (Mps-1) und cdc20. In humanen Zellen existieren zwei Isoformen des MAD2 Proteins, MAD2L1 und MAD2L2 (MAD2B).

Eine de-regulierte Expression von Cyclin E und das Auftreten von Cyclin E Fragmenten führten zu einer Überaktivierung des CDK2/CycE Komplexes und Stimulation des Zellteilungszyklus, was zu der Vermutung führte, dass Patienten mit tumoraler Cyclin E Überexpression mit einer höheren Wahrscheinlichkeit von einer CDK2-gerichteten Therapie profitieren könnten (Hunt, K.K., Keyomarsi, K., Sem. Cancer Biol. 15,319,2005).

Rimkus et al (Int. J. Cancer 120, 207, 2006) beschrieben eine mindestens 3-fach erhöhte Expression von MAD2L2 in 25 von 118 (21%) untersuchten humanen Colonkarzinomproben. Die erhöhte Expression von MAD2L2 korrelierte mit reduzierter Überlebenszeit der Patienten.

Obwohl sich seit mehr als 10 Jahren CDK Inhibitoren in klinscher Entwicklung befinden, wurden bisher noch keine Biomarker beschrieben, die eine Vorhersage des Ansprechens eines Patienten auf die Therapie mit CDK-Inhibitoren erlauben. Solche Stratifikationsmarker erlauben die gezielte Therapie derjenigen Patienten, die mit hoher Wahrscheinlichkeit von einer CDK Inhibitor Therapie profitieren werden. Außerdem erhöht sich die Erfolgswahrscheinlichkeit von klinischen Studien durch Stratifikationsmarker.

WO2010/046035 offenbart besonders effektive pan-CDK-Inhibitoren der Formel (I) 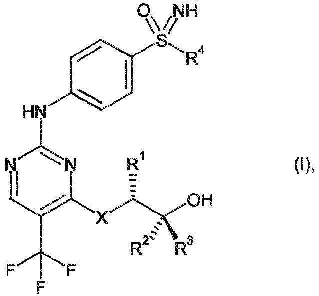 in der
X für -O- oder NH- steht, und
R¹ für eine Methyl-, Ethyl-, Propyl- oder Isopropylgruppe steht, und
R² und R³ unabhängig voneinander für Wasserstoff, eine Methyl- oder Ethylgruppe stehen, und
R⁴ für eine C₁-C₆-Alkylgruppe oder einen C₃-C₇-Cycloalkylring steht,
sowie deren Salze, Diatereomere und Enantiomere,
Der Anmeldung liegen folgende Definitionen zugrunde:

### C₁-C₆-Alkyl

Unter einer C₁-C₆-Alkylgruppe ist jeweils ein geradkettiger oder verzweigter Alkylrest zu verstehen, wie beispielsweise ein Methyl-, Ethyl-, Propyl- Isopropyl-, Butyl-, Isobutyl-, sek. Butyl-, tert. Butyl-, Pentyl-, Isopentyl- oder ein Hexylrest.

### C₃-C₇-Cycloalkyl

Unter einem C₃-C₇-Cycloalkylring ist ein Cyclopropyl- Cyclobutyl-, Cyclopentyl-, Cyclohexyl- oder ein Cycloheptylring zu verstehen.

In der allgemeinen Formel (I) kann X stehen für -O- oder -NH-.
Bevorzugt steht X für -O-.

In der allgemeinen Formel (I) kann R¹ stehen für eine Methyl-, Ethyl-, Propyl- oder Isopropylgruppe. Bevorzugt steht R¹ für eine Methylgruppe.

In der allgemeinen Formel (I) können R² und R³ unabhängig voneinander stehen für Wasserstoff, eine Methyl- oder Ethylgruppe.
Bevorzugt stehen R² und R³ unabhängig voneinander für Wasserstoff oder eine Methylgruppe. Besonders bevorzugt steht R² für eine Methylgruppe und R³ für Wasserstoff oder eine Methylgruppe.

In der allgemeinen Formel (I) kann R⁴ stehen für einen C₁-C₆-Alkylrest oder einen C₃-C₇-Cycloalkylring.
Bevorzugt steht R⁴ für eine Methyl- oder Ethylgruppe oder für einen Cyclopropylring.

Besonders interessant ist die Verbindung (2R,3R)-3-{[2-{[4-(R-Cyclopropylsulfonimidoyl) phenyl]amino}-5-(trifluormethyl)pyrimidin-4-yl]oxy}butan-2-ol (Verbindung A).

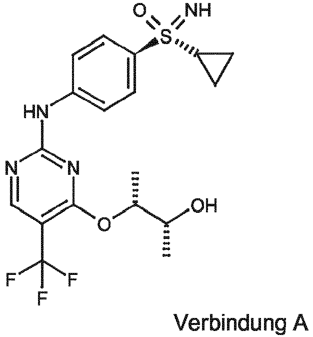

Die Diastereomere der Formel I wurden über eine präparative Chromatographie getrennt. Die experimentellen Details sind in WO2010/046035A1 ausgeführt.

Die Aufgabe der vorliegenden Erfindung ist es, für die pan-CDK-Inhibitoren der WO2010/046035, insbesondere für (2R,3R)-3-{[2-{[4-(S-cyclopropylsulfonimidoyl)phenyl]amino}-5-(trifluormethyl) pyrimidin-4-yl]oxy}butan-2-ol (Verbindung A) einen Stratifikationsmarker zu finden.

Es wurde nun überraschenderweise gefunden, dass MAD2L2 sich als Stratifikationsmarker für humane Brusttumorzellen bei der Behandlung mit den neuen pan-CDK-Inhibitoren der WO2010/046035, insbesondere bei der Behandlung mit der Verbindung A eignet und die Sensitivität vorherzusagen vermag.

Das erfindungsgemäße Verfahren umfasst eine Bestimmung der MAD2L2-Expression als Marker für die Sensitivität von Tumorzellen bzw. von Tumoren gegenüber der Behandlung mit einem CDK-Inhibitor. Vorzugsweise wird hierzu eine quantitative Bestimmung durchgeführt, wobei die Expressionshöhe von MAD2L2 auf Nukleinsäureebene oder/und auf Proteinebene im Tumorgewebe oder in Tumorzellen bestimmt und gegebenenfalls mit der Expressionshöhe im umliegenden Normalgewebe verglichen wird.

Die Expressionshöhe von MAD2L2 kann durch Standardmethoden ermittelt werden. Einer bevorzugten Ausführungsform wird eine Bestimmung auf Nukleinsäureebene, z.B. eine Bestimmung der Transkriptmenge durchgeführt. Quantitative Bestimmungen der MAD2L2-Expression auf Nukleinsäureebene können beispielsweise eine Hybridisierung mit markierten für MAD2L2-spezifischen Sonden, Nukleinsäure-Amplifizierungsreaktionen, Gen-Chip Hybridiesierungen, und/oder Transkript-Sequenzierungen umfassen. Bevorzugte Bestimmungsverfahren sind quantitative PCR oder Realtime PCR. Quantitative Bestimmungen auf Proteinebene können immunologische Nachweisverfahren unter Verwendung von Anti-MAD2L2-Antikörpern beispielsweise im Westernblot oder ELISA-Format umfassen.

Die Probe, in der die MAD2L2-Expression bestimmt werden soll, kann beispielsweise aus einer Zellkultur oder einem Organismus, z.B. einem Säuger, insbesondere einem Menschen, aber auch aus einem Versuchstier stammen. Besonders bevorzugt wird eine Bestimmung an einer Probe durchgeführt, die aus einer Kultur von Tumorzellen, insbesondere von menschlichen Tumorzellen, oder aus einem Tumorpatienten, insbesondere einem menschlichen Patienten oder einem Versuchstier für die Tumorforschung stammt. Die Probe kann aus dem Tumor selbst oder aus abgelösten Tumorzellen, z.B. zirkulierende Tumorzellen aus Körperflüssigkeiten, z.B. Blut, stammen.

In einer bevorzugten Ausführungsform kann das erfindungsgemäße Verfahren zur Therapieauswahl (Therapieentscheidung, Stratifikation) bei der Behandlung eines Patienten, insbesondere eines menschlichen Patienten, im Rahmen eines Therapieverfahrens verwendet werden. Weiterhin kann das erfindungsgemäße Verfahren bei der Behandlung eines Versuchstiers im Rahmen der Identifizierung oder/und Charakterisierung neuer Wirkstoffe dienen. In einer weiteren bevorzugten Ausführungsform kann das Verfahren in einer Zellkultur, beispielsweise im Rahmen von Screening-Prozessen durchgeführt werden.

Das Verfahren umfasst eine oder mehrere Bestimmungen. Vorzugsweise wird vor der erstmaligen Verabreichung des CDK-Inhibitors eine Bestimmung der Expression von MAD2L2 in einer Probe der zu untersuchenden Zellkultur oder des zu untersuchenden Organismus durchgeführt.

### Proliferationsassays

### Methode 1

Dieser Assay wurde für die folgenden Zelllinien verwendet: MCF 10A, SK-BR-3, MCF7, HCT 116, HT-29, SW480, Caco-2, MIAPaCa-2, DU145, PC3, HeLa, Caki2, 786-O, A-375, NCI-H460, NCI-H69, NCI-H1975, A549.
Kultivierte humane Tumorzellen (ursprünglich bezogen von der ATCC, HeLa-MaTu und HeLa-MaTu-ADR, ursprünglich bezogen von der Epo GmbH, Berlin) wurden in einer Dichte von 1000 bis 5000 Zellen/Messpunkt, je nach Wachstumsgeschwindigkeit der Zelllinie, in einer 96-Loch Multititerplatte in 200 µl Wachstumsmedium (DMEM/HAMS F12, 2 mM L-Glutamin,10% Fötales Kälberserum) ausplattiert. Nach 24 Stunden wurden die Zellen einer Platte (Nullpunkt-Platte) mit Kristallviolett gefärbt (s.u.), während das Medium der anderen Platten durch frisches Kulturmedium (200 µl), dem die Testsubstanzen in verschiedenen Konzentrationen (0 µM, sowie im Bereich 0,01 - 30 µM; die finale Konzentration des Lösungsmittels Dimethylsulfoxid betrug 0,5%) zugesetzt waren, ersetzt. Die Zellen wurden für 4 Tage in Anwesenheit der Testsubstanzen inkubiert. Die Zellproliferation wurde durch Färbung der Zellen mit Kristallviolett bestimmt: Die Zellen wurden durch Zugabe von 20 µl/Messpunkt einer 11%igen Glutaraldehyd-Lösung 15 min bei Raumtemperatur fixiert. Nach dreimaligem Waschen der fixierten Zellen mit Wasser wurden die Platten bei Raumtemperatur getrocknet. Die Zellen wurden durch Zugabe von 100 µl/Messpunkt einer 0,1%igen Kristallviolett-Lösung (pH durch Zugabe von Essigsäure auf pH3 eingestellt) gefärbt. Nach dreimaligem Waschen der gefärbten Zellen mit Wasser wurden die Platten bei Raumtemperatur getrocknet. Der Farbstoff wurde durch Zugabe von 100 µl/Messpunkt einer 10%igen Essigsäure-Lösung gelöst. Die Extinktion wurde photometrisch bei einer Wellenlänge von 595 nm bestimmt. Die prozentuale Änderung des Zellwachstums wurde durch Normalisierung der Messwerte auf die Extinktionwerte der Nullpunktplatte (=0%) und die Extinktion der unbehandelten (0 µM) Zellen (=100%) berechnet. Die Messdaten wurden normalisiert auf 0% Inhibition (Zellproliferation ohne Inhibitor) und 100% Inhibition (Nullpunktplatte). Die Bestimmung der IC₅₀ Werte erfolgte mittels eines 4-Parameter Fits unter Benutzung firmeneigener Software.

### Methode 2

Dieser Assay wurde für die folgenden Zelllinien verwendet: KPL-1, MDA-MB-453, Hs 578T, MDA-MB-231, MCF 10A, MDA-MB-468, ZR-75-1, T-47D, MDA-MB-435s, DU-4475, BT-20, BT-474, EVSA-T, BT-549, NCI-H460, NCI-H810, NCI-H441, NCI-H1838, NCI-H69, NCI-H2030, NCI-H358, NCI-H1793, NCI-H1048, SK-MES-1, NCI-H2347, NCI-H1975, A549, NCI-H23, NCI-H2170, NCI-H2228, NCI-H661, NCI-H1703, NCI-H1581, NCI-H226, NCI-H1563, NCI-H522, ChaGo-K-1, NCI-H1437. Die Hemmung der Zellproliferation durch die Verbindung A wurde mittels des Vybrant MTT Cell Proliferation Assays bei der Firma Invitrogen bestimmt.

### Affymetrix Gen-Chip Assay

Dieser Assay wurde zur Bestimmung des relativen mRNA Levels in den verwendeten Tumorzelllinien benutzt.

Kultivierte humane Tumorzellen wurden in mit der gleichen Zellzahl / cm² Plattenfläche in 10 cm Zellkulturschalen ausgesät, wie sie in den Proliferationsassays verwendet wurden, und für 24 Stunden in Wachstumsmedium bei 37°C inkubiert. Danach wurde das Medium abgenommen und die Zellen wurden 2 x mit je 5 ml Phosphat-gepufferter Natriumchlorid-Lösung (PBS) gewaschen. Anschließend wurden die Zellen in 600 µl RLT-Puffer (Qiagen) mit 1% Beta-Mercaptoethanol suspendiert. Die Suspension wurde unter Verwendung eines QIAShredder entsprechend der Vorschrift des Herstellers homogenisiert. Die anschließende RNA Extraktion wurde unter Verwendung des RNeasy Mini Kit (Qiagen) entsprechend der Herstellervorschrift durchgeführt. Weiterhin wurde eine DNase Verdauung unter Verwendung des RNase-freien DNase Kit (Qiagen) entsprechend der Herstellervorschrift durchgeführt.

Die RNA Endkonzentration wurde durch Messung der optischen Dichte bei 260 und 280 nm bestimmt. Zusätzlich wurde eine Qualitätsprüfung der RNA auf einem Agilent Bioanalyzer durchgeführt. Für weitere Analysen wurde nur RNA mit einem Verhältnis von 28S/18S rRNA von mehr als 1,0 verwendet.

5 µg der RNA-Proben wurden zur Synthese von doppelsträngiger cDNA mit dem One-Cycle cDNA Synthesis Kit (Affymetrix) in Gegenwart eines T7-Oligo (dT)₂₄ DNA Oligonukleotideprimer entsprechend der Herstellervorschrift verwendet. Nach der Synthese wurde die cDNA unter Verwendung des Affymetrix GeneChip Sample Cleanup Modul aufgereinigt. Die aufgereinigte cDNA wurde dann unter Verwendung des GeneChip IVT Markierungs-Kit (Affymetrix) in Anwesenheit biotinylierter Ribonukleotide *in vitro* transkribiert, wobei Biotin-markierte cRNA erhalten wurde. Die markierte cRNA wurde dann unter Verwendung des GeneChip Sample Cleanup Modul (Affymetrix) aufgereinigt. Die markierte cRNA wurde durch Messungen der optischen Dichte bei 260 und 280 nm quantitativ bestimmt und einer Qualtitätsüberprüfung auf dem Agilent Bioanalyzer unterzogen. 30 µg markierte cRNA wurden unter Verwendung des Fragmentierungs-Puffers aus dem GeneChip Sample Cleanup Modul (Affymetrix) fragmentiert. Anschließend wurden 10 µg fragmentierte cRNA auf einem Microarray des Typs Human U133 Plus 2.0 (Affymetrix) hybridisiert. Der Array wurde dann gewaschen und mit Streptavidin-R-Phycoerythrin (SAPE, Molecular Probes) markiert. Das Signal wurde unter Verwendung eines biotinylierten Anti-Streptavidin Ziegenantikörpers (Vector Laboratories) gefolgt von einer weiteren Markierung mit SAPE amplifiziert. Die Arrays wurden unter Verwendung der GeneChip Fluidics Station 450 (Affymetrix) markiert. Der Array wurde dann bei 570 nm unter Verwendung eines konfokalen Laserscanners (GeneChip-3000 Scanner, Affymetrix) gescannt und mit der Affymetrix GeneChip Software in quantitative Einzelwerte (je 1 Wert pro Signal, 40 Einzelwerte pro Gen) umgewandelt. Die Einzelwerte wurden unter Verwendung einer Implementierung des Affymetrix MAS5 Algorithmus von Genedata REFINER® zu einem Wert pro Gen zusammengefasst.

Die Prozedur wird unter Verwendung von je drei Microarrays (Replikate) für jede der Zellinien wiederholt. Die resultierenden Einzelwerte aller Gene und Replikate wurden auf den Median aller Werte normiert. Im Anschluss wurde jeder Wert pro Gen und Replikat mittels Berechnung des harmonischen Mittels zu einem Wert pro Gen und Zellinie zusammengefasst. Zwischen den so berechneten mRNA-Expressionswerten und den vorher beschriebenen IC-50 Werten aus den Proliferationsassays wurde der Korrelationskoeffizient nach Pearson zwischen Gen und Testsubstanz jeweils für alle Zellinien berechnet.

Die Verbindung A wurde in den Zelllinien der Tabelle 1 untersucht, die beispielhaft die angegebenen Sub-Indikationen vertreten.

**Tab.1**

| Tumorindikation | Zelllinie |
|---|---|
| Mammakarzinom | KPL-1, MCF 10A, SK-BR-3, MCF7, MDA-MB-453, Hs 578T, MDA-MB-231, MDA-MB-468, ZR-75-1, T-47D, MDA-MB-435s, DU-4475, BT-20, BT-474, EVSA-T, BT-549 |
| Kolonkarzinom | HCT 116, HT-29, SW480, Caco-2 |
| Pankreaskarzinom | MIAPaCa-2 |
| Prostatakarzinom | DU145, PC3 |
| Cervixkarzinom | HeLa |
| Nierenkarzinom | Caki2, 786-O |
| Lungenkarzinom | NCI-H460, NCI-H810, NCI-H441, NCI-H1838, NCI-H69, NCI-H2030, NCI-H358, NCI-H1793, NCI-H1048, SK-MES-1, NCI-H2347, NCI-H1975, A549, NCI-H23, NCI-H2170, NCI-H2228, NCI-H661, NCI-H1703, NCI-H1581, NCI-H226, NCI-H1563, NCI-H522, ChaGo-K-1, NCI-H1437 |
| Melanom | A-375 |

Tabelle 2 listete 62 Gene auf, die Proteine kodieren, die eine regulatorische Funktion im Zellteilungszyklus haben und für die Korrelationsanalyse herangezogen wurden.

**Tab. 2**

| Gen Symbol | Gen-ID | | Kodiertes Protein |
|---|---|---|---|
| CDK1 | 983 | | cyclin-dependent kinase 1 |
| CDK2 | 1017 | | cyclin-dependent kinase 2 |
| CDK3 | 1018 | | cyclin-dependent kinase 3 |
| CDK4 | 1019 | | cyclin-dependent kinase 4 |
| CDK6 | 1021 | | cyclin-dependent kinase 6 |
| CDK7 | 1022 | | cyclin-dependent kinase 7 |
| CDKN1A | 1026 | | cyclin-dependent kinase inhibitor 1A (p21, Cip1) |
| CDKN1B | 1027 | | cyclin-dependent kinase inhibitor 1B (p27, Kip1) |
| CDKN1C | 1028 | | cyclin-dependent kinase inhibitor 1C (p57, Kip2) |
| CDKN2A | 1029 | | cyclin-dependent kinase inhibitor 2A (p 16) |
| CDKN2B | 1030 | | cyclin-dependent kinase inhibitor 2B (p15) |
| CDKN2C | 1031 | | cyclin-dependent kinase inhibitor 2C (p 18) |
| CDKN2D | 1032 | | cyclin-dependent kinase inhibitor 2D (p 19) |
| CDKN3 | 1033 | | cyclin-dependent kinase inhibitor 3 |
| PLK1 | 5347 | | polo-like kinase 1 |
| PLK2 | 10769 | | polo-like kinase 2 |
| PLK3 | 1263 | | polo-like kinase 3 |
| PLK4 | 10733 | | polo-like kinase 4 |
| AURKA | 6790 | | aurora kinase A |
| AURKB | 9212 | | aurora kinase B |
| CHEK1 | 1111 | | CHK1 checkpoint homolog |
| CHEK2 | 11200 | | CHK2 checkpoint homolog |
| CCNA1 | 8900 | | cyclin A1 |
| CCNA2 | 890 | | cyclin A2 |
| CCNB1 | 891 | | cyclin B1 |
| CCNB1IP1 | 57820 | | cyclin B1 interacting protein 1 |
| CCNB2 | 9133 | | cyclin B2 |
| CCNB3 | 85417 | | cyclin B3 |
| CCNC | 892 | | cyclin C |
| CCND1 | 595 | | cyclin D1 |
| CCND2 | 894 | | cyclin D2 |
| CCND3 | 896 | | cyclin D3 |
| CCNDBP1 | 23582 | | cyclin D-type binding-protein 1 |
| CCNE1 | 898 | | cyclin E1 |
| CCNE2 | 9134 | | cyclin E2 |
| CCNF | 899 | | cyclin F |
| CCNG1 | 900 | | cyclin G1 |
| CCNG2 | 901 | | cyclin G2 |
| CCNH | 902 | | cyclin H |
| CCNI | 10983 | | cyclin I |
| CCNI2 | 645121 | | cyclin I family, member 2 |
| CCNJ | 54619 | | cyclin J |
| CCNJL | 79616 | | cyclin J-like |
| CCNK | 8812 | | cyclin K |
| CCNL1 | 57018 | | cyclin L1 |
| CCNL2 | 81669 | | cyclin L2 |
| CCNO | 10309 | | cyclin O |
| CCNT1 | 904 | | cyclin T1 |
| CCNT2 | 905 | | cyclin T2 |
| CCNY | 219771 | | cyclin Y |
| CCNYL1 | 151195 | | cyclin Y-like 1 |
| TTK | 7272 | | TTK protein kinase |
| BUB1 | 699 | | budding uninhibited by benzimidazoles 1 homolog |
| BUB1B | 701 | | budding uninhibited by benzimidazoles 1 homolog beta |
| BUB3 | 9184 | | budding uninhibited by benzimidazoles 3 homolog |
| MAD1L1 | 8379 | | MAD1 mitotic arrest deficient-like 1 |
| MAD2L1 | 4085 | | MAD2 mitotic arrest deficient-like 1 |
| MAD2L1BP | 9587 | | MAD2L1 binding protein |
| MAD2L2 | 10459 | | MAD2 mitotic arrest deficient-like 2 |
| CDC20 | 991 | | cell division cycle 20 homolog |
| CDC20B | 166979 | | cell division cycle 20 homolog B |
| WEE1 | 7465 | | WEE1 homolog |

Tabelle 3 zeigt die Ergebnisse aus den Proliferationsassays.

**Tab.3**

| | Verbindung A |
|---|---|
| | IC50 [nM] |
| Brusttumor-Zelllinien | |
| KPL-1 | 6.44 |
| MCF 10A | 20 |
| SK-BR-3 | 13 |
| MCF7 | 15 |
| MDA-MB-453 | 15.1 |
| Hs 578T | 16.8 |
| MDA-MB-231 | 20.2 |
| MDA-MB-468 | 28.8 |
| ZR-75-1 | 32.5 |
| T-47D | 33.8 |
| MDA-MB-435s | 36.4 |
| DU-4475 | 37.3 |
| BT-20 | 38.1 |
| BT-474 | 42.6 |
| EVSA-T | 45 |
| BT-549 | 84.1 |
| Colonkarzinom-Zelllinien | |
| HCT 116 | 18 |
| HT-29 | 29 |
| SW480 | 15 |
| Caco-2 | 16 |
| Pankreaskarzinom-Zelllinien | |
| MIAPaCa-2 | 21 |
| Prostatakarzinom-Zellinien | |
| DU145 | 8 |
| PC3 | 25 |
| Cervixkarzinom-Zelllinien | |
| HeLa | 12 |
| Nierenkarzinom-Zelllinien | |
| Caki2 | 26 |
| 786-O | 20 |
| Melanom-Zelllinien | |
| A-375 | 14 |
| Lungenkarzinom-Zelllinien | |
| NCI-H460 (nicht-kleinzelliges Lungenkarzinom) | 27 |
| NCI-H810 (nicht-kleinzelliges Lungenkarzinom) | 9.01 |
| NCI-H441 (Papilläres Lungenkarzinom) | 10 |
| NCI-H1838 (nicht-kleinzelliges Lungenkarzinom) | 15.9 |
| NCI-H69 (kleinzelliges Lungenkarzinom) | 27 |
| NCI-H2030 (nicht-kleinzelliges Lungenkarzinom) | 17.7 |
| NCI-H358 (nicht-kleinzelliges Lungenkarzinom) | 19.4 |
| NCI-H1793 (nicht-kleinzelliges Lungenkarzinom) | 22.5 |
| NCI-H1048 (kleinzelliges Lungenkarzinom) | 25 |
| SK-MES-1 (Plattenzellkarzinom) | 26.5 |
| NCI-H2347 (nicht-kleinzelliges Lungenkarzinom) | 28 |
| NCI-H1975 (nicht-kleinzelliges Lungenkarzinom) | 24 |
| A549 (nicht-kleinzelliges Lungenkarzinom) | 31 |
| NCI-H23 (nicht-kleinzelliges Lungenkarzinom) | 45.4 |
| NCI-H2170 (kleinzelliges Lungenkarzinom) | 48.7 |
| NCI-H2228 (nicht-kleinzelliges Lungenkarzinom) | 52.1 |
| NCI-H661 (nicht-kleinzelliges Lungenkarzinom) | 53.1 |
| NCI-H1703 (nicht-kleinzelliges Lungenkarzinom) | 53.6 |
| NCI-H1581 (nicht-kleinzelliges Lungenkarzinom) | 53.8 |
| NCI-H226 (Mesotheliom) | 54.6 |
| NCI-H1563 (nicht-kleinzelliges Lungenkarzinom) | 59.1 |
| NCI-H522 (nicht-kleinzelliges Lungenkarzinom) | 65.4 |
| ChaGo-K-1 (undifferenziertes Bronchialkarzinom) | 69.4 |
| NCI-H1437 (nicht-kleinzelliges Lungenkarzinom) | 69.9 |

Tabelle 4 zeigt die relativen mRNA Mengen der 62 Zellzyklus-regulatorischen Gene in den 51 untersuchten Zelllinien ermittelt in Affymetrix Gene-Chip Hybridisierungsstudien.

**Tab. 4**

| | **AURKA** | **AURKB** | **BUB1B** | **BUB1** | **BUB3** | **CCNA1** | **CCNA2** | **CCNB1IP1** | **CCNB1** | **CCNB2** | **CCNB3** | **CCNC** | **CCND1** | **CCND2** | **CCNDBP1** |
|---|---|---|---|---|---|---|---|---|---|---|---|---|---|---|---|
| KPL1 | 60087 | 19709 | 13265 | 10880 | 78630 | 491 | 18335 | 17308 | 40182 | 33709 | 708 | 20839 | 48200 | 654 | 11047 |
| MCF10A | 34913 | 8307 | 23706 | 14238 | 135711 | 6835 | 28358 | 42206 | 89275 | 53637 | 1021 | 125110 | 27295 | 25935 | 41515 |
| SK-BR-3 | 127962 | 7888 | 33366 | 18758 | 114446 | 4324 | 41783 | 54014 | 99234 | 62103 | 1177 | 89742 | 25227 | 639 | 23071 |
| MCF7 | 66402 | 16299 | 19440 | 13970 | 114589 | 893 | 21914 | 44705 | 82679 | 41645 | 938 | 85000 | 76589 | 381 | 17308 |
| MDAMB453 | 35610 | 6310 | 44008 | 12952 | 163447 | 338 | 32659 | 19811 | 75501 | 86143 | 1012 | 83630 | 9022 | 556 | 10963 |
| HS578T | 52223 | 28323 | 50402 | 21942 | 171634 | 189 | 33270 | 39922 | 97045 | 75996 | 544 | 69587 | 52776 | 160 | 39611 |
| MDAMB231 | 132277 | 46600 | 65257 | 63651 | 244291 | 2767 | 77385 | 36196 | 204466 | 95832 | 1552 | 90089 | 85794 | 689 | 41418 |
| MDAMB468 | 73003 | 30792 | 53846 | 39361 | 175379 | 772 | 54628 | 86879 | 163813 | 103370 | 1046 | 208429 | 19589 | 101 | 22200 |
| ZR751 | 73014 | 15433 | 35067 | 21664 | 137095 | 1018 | 38687 | 37929 | 107878 | 77971 | 1666 | 101304 | 62178 | 140 | 11607 |
| T47D | 79613 | 14780 | 53655 | 19656 | 119998 | 280 | 45386 | 15532 | 185519 | 115028 | 1913 | 66247 | 21505 | 464 | 14895 |
| MDAMB435S | 99910 | 22673 | 68619 | 37445 | 225787 | 108 | 40529 | 52837 | 179811 | 159076 | 1262 | 79039 | 69604 | 268 | 28254 |
| DU4475 | 27257 | 17877 | 31276 | 22899 | 132503 | 361 | 26780 | 11136 | 61736 | 52202 | 1126 | 13965 | 5153 | 484 | 22204 |
| BT20 | 59155 | 16032 | 35465 | 19235 | 54212 | 967 | 32077 | 22275 | 75663 | 67015 | 5004 | 59513 | 25778 | 962 | 23759 |
| BT474 | 95568 | 8851 | 27409 | 12488 | 61323 | 734 | 18134 | 67693 | 46558 | 45940 | 1226 | 60702 | 25565 | 463 | 9236 |
| EVSAT | 132254 | 9140 | 27190 | 11748 | 168616 | 469 | 31774 | 20491 | 84263 | 57295 | 1146 | 171163 | 8719 | 161 | 19286 |
| BT549 | 104992 | 30879 | 46425 | 28643 | 146073 | 4155 | 45392 | 40775 | 172233 | 41266 | 1046 | 128212 | 9447 | 335 | 2266 |
| HCT116 | 66028 | 36907 | 87813 | 45667 | 273237 | 272 | 49299 | 64683 | 164167 | 94782 | 2284 | 136271 | 71527 | 209 | 30507 |
| HT29 | 91034 | 22421 | 55594 | 35853 | 163773 | 463 | 55007 | 45582 | 166751 | 116312 | 1009 | 117943 | 15940 | 238 | 32229 |
| SW480 | 72796 | 35221 | 37225 | 36785 | 119547 | 161 | 40022 | 29231 | 112696 | 85041 | 1102 | 93170 | 198818 | 13570 | 26414 |
| CACO2 | 174500 | 37352 | 52965 | 54378 | 149417 | 276 | 82241 | 78729 | 243245 | 133942 | 1044 | 194111 | 43688 | 37183 | 29741 |
| MIAPACA2 | 74575 | 33355 | 44046 | 43296 | 148303 | 267 | 34959 | 34158 | 169941 | 95169 | 1203 | 123640 | 19765 | 764 | 20678 |
| DU145 | 73547 | 21613 | 55396 | 30225 | 177577 | 3343 | 25582 | 41263 | 111798 | 73889 | 2636 | 86219 | 34482 | 267 | 44471 |
| PC3 | 61659 | 12697 | 55837 | 36970 | 159589 | 5657 | 23942 | 27195 | 100313 | 48892 | 722 | 72154 | 68926 | 307 | 23286 |
| HELA | 74457 | 21496 | 73701 | 22733 | 202199 | 563 | 52633 | 29953 | 158606 | 102931 | 855 | 95815 | 10189 | 339 | 22054 |
| Caki2 | 45628 | 5675 | 39081 | 29085 | 90789 | 6407 | 35447 | 15429 | *65040* | 42794 | 1479 | 60949 | 24040 | 432 | 14974 |
| 7860 | 77549 | 26209 | 49205 | 43467 | 151043 | 3033 | 47113 | 34886 | 103593 | 82152 | 615 | 105183 | 68088 | 357 | 28043 |
| A375 | 43673 | 20524 . | 34099 | 15089 | 141130 | 4119 | 26523 | 18131 | 90572 | 50907 | 1203 | 46133 | 60715 | 734 | 18184 |
| NCIH226 | 18987 | 4771 | 5859 | 7639 | 37827 | 1040 | 13846 | 16170 | 26358 | 32041 | 1006 | 42815 | 30528 | 4643 | 11841 |
| NCIH460 | 89625 | 17579 | 32287 | 25536 | 132449 | 247 | 29198 | 56785 | 108118 | 52566 | 1986 | 68165 | 10759 | 177 | 18456 |
| NCIH810 | 53140 | 13687 | 32901 | 16399 | 54233 | 637 | 27197 | 44593 | 49505 | 52117 | 676 | 44269 | 2435 | 623 | 28322 |
| NCIH1838 | 32887 | 19843 | 32004 | 14252 | 38809 | 293 | 14462 | 21164 | 38211 | 37795 | 1988 | 31341 | 44366 | 656 | 23656 |
| NCIH2030 | 69179 | 25719 | 50663 | 31075 | 76930 | 1439 | 34599 | 13296 | 64336 | 44768 | 2225 | 30516 | 15321 | 668 | 24893 |
| NCIH358 | 7896 | 3918 | 9704 | 5131 | 26780 | 545 | 6113 | 31100 | 12239 | 15280 | 2986 | 19233 | 35286 | 319 | 11794 |
| NCIH1793 | 31869 | 5416 | 26253 | 10386 | 39172 | 1148 | 18832 | 13474 | 38759 | 25575 | 5233 | 23796 | 16101 | 703 | 11431 |
| NCIH2347 | 38717 | 10405 | 18367 | 16974 | 53179 | 730 | 27524 | 22373 | 81585 | 32726 | 1850 | 38105 | 76835 | 2645 | 21251 |
| NCIH1975 | 24095 | 10956 | 13161 | 8853 | 50520 | 3301 | 13028 | 19691 | 36185 | 17669 | 1812 | 30408 | 22628 | 919 | 18635 |
| A549 | 85072 | 36346 | 40617 | 29470 | 145586 | 268 | 37058 | 28256 | 157943 | 98461 | 1233 | 78118 | 43552 | 278 | 20600 |
| NCIH23 | 3481 | 1041 | 1904 | 734 | 34821 | 554 | 1983 | 12402 | 2278 | 2392 | 940 | 22242 | 22776 | 1077 | 26398 |
| NCIH2228 | 10688 | 1429 | 8638 | 5243 | 35617 | 3023 | 9781 | 17870 | 24662 | 10757 | 1110 | 24504 | 24771 | 4302 | 20331 |
| NCIH661 | 29728 | 12309 | 17537 | 12839 | 25077 | 722 | 36211 | 21546 | 23425 | 24205 | 4548 | 24684 | 3656 | 883 | 12882 |
| NCIH1703 | 41730 | 19655 | 38478 | 18648 | 59750 | 1601 | 36182 | 14451 | 79569 | 41934 | 8474 | 49850 | 6373 | 92426 | 15653 |
| NCIH1581 | 30583 | 13348 | 19860 | 15472 | 61848 | 1056 | 21693 | 18094 | 34744 | 21939 | 3052 | 38088 | 22083 | 59479 | 6609 |
| NCIH1563 | 33598 | 2063 | 8042 | 3942 | 34433 | 693 | 11431 | 17888 | 18305 | 10696 | 2026 | 39449 | 17891 | 875 | 16138 |
| NCIH522 | 22431 | 6567 | 17392 | 12945 | 39954 | 701 | 18687 | 9338 | 24099 | 15579 | 2037 | 20182 | 9779 | 606 | 5687 |
| NCIH1437 | 29997 | 8161 | 29979 | 11391 | 64269 | 1164 | 20668 | 11982 | 47843 | 30182 | 1162 | 26696 | 36427 | 735 | 8907 |
| NCIH441 | 35979 | 17896 | 28958 | 15971 | 57455 | 490 | 24281 | 26814 | 50870 | 33166 | 952 | 32001 | 37893 | 702 | 23983 |
| NCIH69 | 14587 | 9410 | 28863 | 8578 | 40234 | 9193 | 20343 | 30090 | 51248 | 40047 | 1320 | 21218 | 975 | 501 | 5518 |
| NCIH1048 | 32538 | 9479 | 39895 | 12935 | 76698 | 1937 | 37710 | 15422 | 53259 | 36826 | 3074 | 37329 | 8030 | 753 | 20986 |
| NCIH2170 | 24942 | 7775 | 27835 | 7596 | 30037 | 410 | 31153 | 13755 | 31099 | 20680 | 1318 | 46194 | 22041 | 582 | 9402 |
| SKMES1 | 33277 | 7855 | 27593 | 12245 | 46034 | 594 | 32503 | 12895 | 39311 | 28313 | 1564 | 45079 | 48918 | 858 | 20602 |
| CHAGOK1 | 55008 | 9057 | 30223 | 10735 | 26993 | 871 | 43567 | 18209 | 42782 | 32033 | 1481 | 34545 | 10716 | 1972 | 11709 |

| | **CCNE1** | **CCNE2** | **CCNF** | **CCNG1** | **CCNG2** | **CCNH** | **CCNI** | **CNJL** | **CCNJ** | **CCNK** | **CCNL1** | **CCNL2** | **CCNO** | **CCNT1** | **CCNT2** |
|---|---|---|---|---|---|---|---|---|---|---|---|---|---|---|---|
| KPL1 | 7449 | 10202 | 8871 | 51799 | 10803 | 21255 | 62025 | 495 | 2582 | 28501 | 14840 | 23740 | 4545 | 3389 | 3559 |
| MCF10A | 5870 | 8917 | 6513 | 214311 | 46371 | 36521 | 116413 | 881 | 3918 | 19601 | 19360 | 43684 | 3926 | 3250 | 9435 |
| SK-BR-3 | 11395 | 9917 | 11550 | 40298 | 25045 | 25747 | 93707 | 552 | 1630 | 11365 | 10480 | 21899 | 1443 | 2279 | 5827 |
| MCF7 | 7982 | 27985 | 9084 | 114479 | 37286 | 38805 | 120523 | 1421 | 6153 | 41185 | 19297 | 22037 | 2471 | 3104 | 9067 |
| MDAMB453 | 3902 | 35004 | 12953 | 76736 | 63267 | 25735 | 109657 | 275 | 3102 | 23207 | 13118 | 18166 | 13179 | 814 | 9630 |
| HS578T | 9497 | 30371 | 7353 | 122062 | 42063 | 44285 | 112047 | 276 | 3067 | 18921 | 23818 | 37708 | 2371 | 2046 | 6298 |
| MDAMB231 | 12610 | 46334 | 10656 | 71751 | 24820 | 57429 | 102215 | 4203 | 6713 | 24617 | 12932 | 23517 | 4387 | 2164 | 6686 |
| MDAMB468 | 14018 | 32314 | 16590 | 200683 | 16788 | 45188 | 110454 | 2233 | 4752 | 27090 | 19304 | 16337 | 2769 | 1810 | 7644 |
| ZR751 | 7913 | 33222 | 10848 | 120001 | 48045 | 37453 | 126774 | 773 | 4561 | 30143 | 20407 | 22858 | 2196 | 2661 | 15250 |
| T47D | 5209 | 51290 | 10496 | 106730 | 23705 | 53287 | 66332 | 1562 | 6148 | 30258 | 15232 | 18540 | 9622 | 3401 | 7187 |
| MDAMB435S | 9911 | 26574 | 9343 | 76112 | 7885 | 69644 | 45102 | 1937 | 2488 | 17254 | 26770 | 11611 | 9103 | 2255 | 5126 |
| DU4475 | 12821 | 22050 | 10181 | 67147 | 5496 | 8263 | 16196 | 722 | 3048 | 11491 | 14112 | 12759 | 4848 | 2824 | 6297 |
| BT20 | 8064 | 20885 | 7976 | 20197 | 8817 | 20775 | 66365 | 241 | 1525 | 21944 | 10685 | 11214 | 3110 | 2559 | 5657 |
| BT474 | 3386 | 37458 | 8096 | 42635 | 91312 | 8382 | 160602 | 2035 | 6084 | 19793 | 14258 | 15422 | 1272 | 2314 | 9358 |
| EVSAT | 8746 | 89382 | 9298 | 80607 | 9277 | 19844 | 81986 | 529 | 2553 | 27309 | 10448 | 25481 | 4584 | 3189 | 8913 |
| BT549 | 17171 | 111778 | 7348 | 69766 | 12548 | 57541 | 78890 | 667 | 8601 | 24518 | 13633 | 23912 | 945 | 1247 | 4265 |
| HCT116 | 25789 | 31245 | 17540 | 191267 | 13514 | 63028 | 96242 | 1472 | 9666 | 33717 | 24582 | 28895 | 7116 | 3789 | 9434 |
| HT29 | 12079 | 16979 | 11710 | 172609 | 17408 | 59099 | 104601 | 1864 | 6640 | 21458 | 14890 | 24108 | 4225 | 2654 | 10445 |
| SW480 | 5985 | 19425 | 8542 | 98231 | 13603 | 38635 | 69164 | 2336 | 5009 | 27180 | 27006 | 29740 | 4290 | 1871 | 7659 |
| CACO2 | 20296 | 19464 | 15387 | 244602 | 9948 | 62280 | 82075 | 3075 | 12189 | 33688 | 23952 | 25959 | 3639 | 4006 | 15584 |
| MIAPACA2 | 16987 | 12615 | 10311 | 168313 | 7779 | 73449 | 86675 | 689 | 5850 | 26089 | 25892 | 35065 | 5496 | 2643 | 8573 |
| DU145 | 7817 | 20946 | 5910 | 77921 | 7782 | 58070 | 85762 | 1967 | 4747 | 28433 | 22827 | 24339 | 3667 | 2729 | 6938 |
| PC3 | 4748 | 26708 | 4891 | 78028 | 13221 | 87461 | 54438 | 2358 | 4231 | 21539 | 17765 | 39738 | 3323 | 2904 | 7894 |
| HELA | 10308 | 23960 | 11588 | 110737 | 12017 | 66020 | 71595 | 1688 | 3268 | 18645 | 11450 | 29199 | 4219 | 2729 | 7807 |
| Caki2 | 8784 | 11005 | 5882 | 96877 | 17575 | 15795 | 65311 | 1572 | 2346 | 15165 | 10863 | 14418 | 2892 | 1270 | 4203 |
| 7860 | 6578 | 27305 | 10993 | 106597 | 14078 | 37659 | 117412 | 1138 | 5194 | 22106 | 13183 | 28445 | 2692 | 3506 | 8151 |
| A375 | 6802 | 16588 | 7715 | 72688 | 4234 | 45503 | 42682 | 2150 | 5311 | 14694 | 13970 | 18289 | 5070 | 1861 | 2947 |
| NCIH226 | 2092 | 3712 | 4330 | 49097 | 4425 | 21703 | 52648 | 203 | 1367 | 12448 | 12322 | 15985 | 3093 | 2494 | 4019 |
| NCIH460 | 7347 | 14398 | 5507 | 157712 | 4716 | 54116 | 63949 | 1931 | 5807 | 21874 | 11758 | 16817 | 4538 | 3205 | 10418 |
| NCIH810 | 79712 | 10930 | 20114 | 22510 | 12404 | 26656 | 86840 | 1344 | 11165 | 21196 | 16688 | 14667 | 1030 | 2892 | 6702 |
| NCIH1838 | 4239 | 8895 | 4221 | 23900 | 12462 | 14388 | 56493 | 1521 | 3432 | 17065 | 10212 | 12636 | 5654 | 1175 | 1843 |
| NCIH2030 | 10546 | 8709 | 6722 | 23169 | 20622 | 34637 | 72705 | 984 | 2391 | 22097 | 11908 | 14229 | 1068 | 2045 | 5731 |
| NCIH358 | 1256 | 2504 | 1835 | 13800 | 25035 | 16565 | 69362 | 1578 | 1863 | 10432 | 8346 | 19168 | 2115 | 1161 | 2958 |
| NCIH1793 | 6466 | 10129 | 3011 | 32761 | 10503 | 27326 | 59685 | 2999 | 2764 | 15195 | 10387 | 5361 | 4420 | 1668 | 2284 |
| NCIH2347 | 8457 | 14631 | 5112 | 41312 | 48651 | 26550 | 87779 | 2999 | 3308 | 21535 | 11444 | 11349 | 4468 | 2575 | 3745 |
| NCIH1975 | 4049 | 4237 | 3443 | 30031 | 16911 | 32740 | 55462 | 1033 | 3053 | 20000 | 6624 | 9230 | 1288 | 1199 | 4793 |
| A549 | 17739 | 10115 | 9795 | 102327 | 4658 | 45722 | 53411 | 2739 | 6158 | 27901 | 15024 | 15727 | 3351 | 2510 | 6088 |
| NCIH23 | 3118 | 772 | 1396 | 51588 | 6894 | 20820 | 84235 | 2319 | 4805 | 11906 | 17412 | 26304 | 7122 | 1050 | 6971 |
| NCIH2228 | 7369 | 5549 | 2463 | 29830 | 6497 | 42934 | 78040 | 6555 | 4769 | 26117 | 11200 | 17110 | 4965 | 2814 | 5968 |
| NCIH661 | 7143 | 1815 | 5024 | 21321 | 7234 | 5650 | 51006 | 1229 | 2787 | 11568 | 5807 | 12103 | 3402 | 1571 | 4718 |
| NCIH1703 | 6553 | 16143 | 6937 | 60337 | 9326 | 27072 | 71597 | 1105 | 5361 | 20939 | 9632 | 21355 | 4100 | 1253 | 5248 |
| NCIH1581 | 8770 | 7106 | 6765 | 20828 | 3270 | 11822 | 56179 | 557 | 7318 | 30563 | 7059 | 17103 | 1121 | 1978 | 5716 |
| NCIH1563 | 9361 | 9513 | 3375 | 45050 | 25317 | 23017 | 62919 | 2298 | 2366 | 13930 | 8030 | 6496 | 1535 | 526 | 6381 |
| NCIH522 | 23722 | 12804 | 4506 | 14699 | 3388 | 10975 | 50119 | 1121 | 5797 | 27379 | 7684 | 11692 | 3590 | 1162 | 4534 |
| NCIH1437 | 6992 | 16572 | 6736 | 16872 | 2742 | 19023 | 62323 | 3196 | 3954 | 24478 | 10165 | 8057 | 4820 | 1669 | 4352 |
| NCIH441 | 5605 | 15168 | 7040 | 38753 | 47990 | 17369 | 80164 | 3972 | 2637 | 19322 | 10797 | 27111 | 2112 | 1581 | 5581 |
| NCIH69 | 7943 | 31851 | 9302 | 25182 | 6495 | 43103 | 52174 | 1166 | 4430 | 10509 | 5265 | 7996 | 15777 | 910 | 2368 |
| NCIH1048 | 16545 | 23224 | 5268 | 37928 | 26799 | 25356 | 75506 | 1868 | 5855 | 16966 | 7060 | 12469 | 3591 | 1626 | 5823 |
| NCIH2170 | 9603 | 9859 | 3955 | 29639 | 9605 | 18626 | 85821 | 1173 | 4716 | 16668 | 9156 | 7773 | 6297 | 1177 | 6888 |
| SKMES1 | 3782 | 7070 | 5691 | 22203 | 16785 | 24370 | 73256 | 3022 | 2729 | 21037 | 10514 | 15374 | 4872 | 918 | 3838 |
| CHAGOK1 | 19286 | 6716 | 6549 | 27293 | 11383 | 12891 | 62833 | 722 | 117 | 16927 | 8711 | 23642 | 5816 | 18796 | 4704 |

| | **CCNYL1** | **CCNY** | **CDC20B** | **CDC20** | **CDK1** | **CDK2** | **CDK3** | **CDK4** | **CDK6** | **CDK7** | **CDKN1A** | **CDKN1B** | **CDKN1C** | **CDKN2A** | **CDKN2B** |
|---|---|---|---|---|---|---|---|---|---|---|---|---|---|---|---|
| KPL1 | 4381 | 8952 | 618 | 33235 | 40424 | 30789 | 2204 | 63959 | 11946 | 16233 | 17886 | 22503 | 627 | 2195 | 1521 |
| MCF10A | 22824 | 26926 | 245 | 35859 | 59001 | 16264 | 3729 | 66301 | 42299 | 38197 | 48775 | 29848 | 12411 | 941 | 49 |
| SK-BR-3 | 4516 | 25848 | 300 | 54435 | 144149 | 22037 | 5111 | 72053 | 6951 | 31424 | 3404 | 42190 | 463 | 15080 | 5868 |
| MCF7 | 8307 | 21784 | 564 | 22827 | 53922 | 34536 | 4038 | 118657 | 3580 | 33265 | 73843 | 74835 | 1137 | 970 | 3597 |
| MDAMB453 | 9161 | 12474 | 642 | 34004 | 86036 | 24896 | 2719 | 81076 | 643 | 17398 | 1407 | 81115 | 2940 | 7086 | 211 |
| HS578T | 33991 | 38961 | 353 | 79236 | 113081 | 27550 | 2579 | 136997 | 66662 | 28058 | 19024 | 46542 | 1533 | 1324 | 16642 |
| MDAMB231 | 20364 | 34496 | 429 | 89770 | 243209 | 43589 | 3563 | 96622 | 40513 | 53269 | 26918 | 47734 | 9120 | 577 | 131 |
| MDAMB468 | 32925 | 15331 | 125 | 183758 | 246851 | 17121 | 6169 | 64686 | 17174 | 31187 | 7811 | 29242 | 2736 | 83704 | 8877 |
| ZR751 | 8331 | 29683 | 317 | 27388 | 134235 | 23529 | 6044 | 140821 | 189 | 39275 | 40616 | 87423 | 485 | 3393 | 1155 |
| T47D | 5114 | 26514 | 3072 | 45820 | 202034 | 41399 | 5669 | 92379 | 1395 | 39341 | 11414 | 29061 | 1361 | 28830 | 3927 |
| MDAMB435S | 17920 | 25082 | 516 | 75099 | 117373 | 25968 | 3979 | 133857 | 22662 | 39980 | 6012 | 16144 | 4805 | 77563 | 2651 |
| DU4475 | 4892 | 13443 | 1005 | 47589 | 84856 | 23132 | 1218 | 53383 | 49060 | 19713 | 12833 | 9337 | 40158 | 12537 | 505 |
| BT20 | 14106 | 18946 | 1012 | 40625 | 72243 | 30606 | 4721 | 46717 | 4992 | 10775 | 4357 | 42073 | 2480 | 1476 | 77 |
| BT474 | 2214 | 14111 | 471 | 31884 | 53753 | 27230 | 3984 | 74764 | 2401 | 15440 | 3156 | 60831 | 2520 | 5253 | 304 |
| EVSAT | 12778 | 27286 | 443 | 29702 | 92306 | 36609 | 4526 | 125415 | 85137 | 14140 | 3073 | 56488 | 1183 | 10447 | 1283 |
| BT549 | 5762 | 34120 | 423 | 84470 | 22109 | 47654 | 2219 | 152880 | 27694 | 35473 | 8706 | 40908 | 1179 | 281653 | 6614 |
| HCT116 | 11126 | 36816 | 236 | 120371 | 208297 | 72131 | 3398 | 168971 | 37281 | 59164 | 54992 | 44485 | 9732 | 35122 | 2066 |
| HT29 | 19596 | 21710 | 424 | 58384 | 163524 | 39319 | 4528 | 130277 | 42756 | 43029 | 9195 | 39008 | 2614 | 18519 | 1281 |
| SW480 | 17271 | 19031 | 402 | 59401 | 91434 | 40373 | 3938 | 108253 | 33414 | 30312 | 21191 | 47872 | 12401 | 27809 | 3967 |
| CACO2 | 54684 | 38499 | 496 | 62658 | 312205 | 51182 | 4403 | 167659 | 61924 | 64311 | 4098 | 122817 | 8105 | 19216 | 1397 |
| MIAPACA2 | 9339 | 25666 | 484 | 96197 | 85663 | 25787 | 4845 | 99734 | 14593 | 43369 | 5089 | 56844 | 2133 | 956 | 118 |
| DU145 | 29595 | 22511 | 460 | 54735 | 114283 | 45604 | 4465 | 70300 | 5042 | 33965 | 16718 | 52334 | 3918 | 212852 | 7490 |
| PC3 | 20138 | 56121 | 260 | 48606 | 170006 | 27756 | 5063 | 95333 | 22119 | 16253 | 9207 | 22520 | 5096 | 65026 | 11397 |
| HELA | 13064 | 15770 | 467 | 67694 | 132487 | 54138 | 2914 | 102373 | 52799 | 26141 | 16508 | 70677 | 21876 | 269053 | 18919 |
| Caki2 | 39002 | 11400 | 726 | 34668 | 72476 | 28952 | 3832 | 90877 | 32888 | 12960 | 34120 | 31731 | 2094 | 2422 | 48 |
| 7860 | 28505 | 32078 | 705 | 93204 | 175500 | 30449 | 4491 | 131095 | 113604 | 15712 | 40389 | 50535 | 2067 | 978 | 49 |
| A375 | 6063 | 15591 | 230 | 68098 | 55382 | 38886 | 3910 | 106783 | 52547 | 26831 | 18389 | 20029 | 3202 | 3200 | 69 |
| NCIH226 | 33613 | 9383 | 1052 | 16134 | 22521 | 25029 | 3017 | 63673 | 40535 | 14649 | 46497 | 16403 | 7021 | 3020 | 5733 |
| NCIH460 | 46119 | 10738 | 377 | 31250 | 118959 | 20679 | 3457 | 99639 | 31260 | 40611 | 16965 | 19523 | 520 | 940 | 82 |
| NCIH810 | 3909 | 19495 | 512 | 53693 | 45896 | 28197 | 4596 | 78474 | 14729 | 10384 | 1064 | 39299 | 17439 | 42443 | 651 |
| NCIH1838 | 12699 | 11087 | 916 | 22319 | 23503 | 16031 | 3673 | 78950 | 8906 | 8703 | 9049 | 24938 | 11398 | 1232 | 22 |
| NCIH2030 | 31179 | 8922 | 803 | 56552 | 43601 | 43962 | 4754 | 52759 | 22531 | 15302 | 14066 | 21265 | 3391 | 20932 | 1865 |
| NCIH358 | 5535 | 7668 | 510 | 7952 | 5090 | 12091 | 2225 | 43895 | 7823 | 9948 | 18077 | 14809 | 4313 | 21755 | 8232 |
| NCIH1793 | 4702 | 5693 | 1072 | 18225 | 28882 | 23275 | 4238 | 58423 | 11879 | 12608 | 7921 | 14837 | 1707 | 1549 | 21194 |
| NCIH2347 | 8619 | 11917 | 728 | 35534 | 32441 | 25349 | 5335 | 60657 | 9168 | 32867 | 22664 | 27749 | 10207 | 25842 | 7015 |
| NCIH1975 | 11612 | 7709 | 735 | 18974 | 17172 | 16886 | 5056 | 59398 | 3716 | 18739 | 6270 | 22548 | 13419 | 9295 | 3985 |
| A549 | 23016 | 21726 | 543 | 55076 | 135093 | 30920 | 4949 | 112191 | 35280 | 34367 | 31946 | 32216 | 1112 | 1146 | 95 |
| NCIH23 | 4340 | 19972 | 820 | 1252 | 1749 | 9152 | 6186 | 54922 | 15207 | 12427 | 1089 | 21061 | 5559 | 24470 | 9133 |
| NCIH2228 | 11660 | 11961 | 933 | 5327 | 8342 | 11627 | 5361 | 62607 | 955 | 16784 | 15211 | 30886 | 17106 | 1452 | 32 |
| NCIH661 | 30038 | 12032 | 1031 | 21485 | 16109 | 20143 | 4672 | 53652 | 15138 | 7877 | 4228 | 13023 | 5662 | 17914 | 1549 |
| NCIH1703 | 47804 | 14061 | 769 | 52930 | 47433 | 46288 | 7827 | 65652 | 12605 | 20916 | 3841 | 40597 | 2583 | 44867 | 2044 |
| NCIH1581 | 10675 | 15903 | 777 | 29826 | 29103 | 31977 | 8731 | 81174 | 18862 | 7804 | 3113 | 21542 | 6209 | 20216 | 1005 |
| NCIH1563 | 5829 | 11675 | 1111 | 11126 | 15374 | 11776 | 4762 | 30387 | 11549 | 9263 | 37245 | 22152 | 6741 | 1830 | 49 |
| NCIH522 | 8089 | 7826 | 959 | 31058 | 19561 | 29262 | 4532 | 54728 | 293 | 7803 | 11968 | 5846 | 1975 | 80732 | 4494 |
| NCIH1437 | 12776 | 13836 | 978 | 18398 | 50832 | 32876 | 7254 | 53515 | 5346 | 12071 | 4730 | 24149 | 1538 | 1215 | 14 |
| NCIH441 | 18398 | 17188 | 518 | 31378 | 34901 | 23962 | 4971 | 49473 | 9511 | 13934 | 10841 | 34381 | 3838 | 10589 | 2317 |
| NCIH69 | 2065 | 8741 | 2025 | 9531 | 24613 | 29635 | 4387 | 62350 | 8083 | 7868 | 45686 | 23232 | 3708 | 44704 | 1288 |
| NCIH1048 | 3243 | 9072 | 604 | 36219 | 43358 | 40307 | 5162 | 44135 | 6357 | 9158 | 14920 | 33676 | 18523 | 61651 | 8772 |
| NCIH2170 | 10278 | 8593 | 907 | 16078 | 39879 | 12108 | 2316 | 33518 | 14734 | 7986 | 3099 | 37263 | 512 | 2025 | 29 |
| SKMES1 | 11376 | 11238 | 711 | 29731 | 24094 | 17433 | 5260 | 41812 | 11924 | 11796 | 12121 | 19711 | 8291 | 1067 | 52 |
| CHAGOK1 | 7092 | 18339 | 591 | 33236 | 3856 | 21646 | 6313 | 31918 | 17577 | 18319 | 2154 | 27678 | 5405 | 12925 | 1386 |

| | **CDKN2C** | **CDKN2D** | **CDKN3** | **CHEK1** | **CHEK2** | **MAD1L1** | **MAD2L1BP** | **MAD2L1** | **MAD2L2** | **PLK1** | **PLK2** | **PLK3** | **PLK4** | **TTK** | **WEE1** |
|---|---|---|---|---|---|---|---|---|---|---|---|---|---|---|---|
| KPL1 | 5301 | 592 | 33679 | 4449 | 1632 | 4841 | 8948 | 21176 | 14196 | 13342 | 70636 | 2389 | 7400 | 8079 | 9022 |
| MCF10A | 7953 | 886 | 30518 | 17467 | 7251 | 3052 | 17355 | 56760 | 12673 | 10151 | 40414 | 3521 | 8129 | 18357 | 25568 |
| SK-BR-3 | 50475 | 1758 | 72086 | 14959 | 15066 | 5870 | 14552 | 84768 | 15033 | 14681 | 5745 | 1434 | 11059 | 27120 | 53706 |
| MCF7 | 10087 | 1026 | 48465 | 11110 | 8704 | 5092 | 20952 | 69586 | 16809 | 11125 | 35947 | 1073 | 10308 | 16215 | 20028 |
| MDAMB453 | 21494 | 3350 | 71765 | 20120 | 13517 | 3215 | 16317 | 90782 | 17475 | 16154 | 51545 | 967 | 15148 | 18763 | 18784 |
| HS578T | 95283 | 5973 | 59812 | 24882 | 8391 | 28612 | 28412 | 63817 | 44555 | 16739 | 57018 | 2159 | 14428 | 33352 | 32375 |
| MDAMB231 | 53073 | 14064 | 128476 | 33045 | 6317 | 12959 | 29722 | 134623 | 28981 | 23060 | 133894 | 1810 | 33258 | 46790 | 48909 |
| MDAMB468 | 44520 | 16944 | 97918 | 48012 | 12291 | 14557 | 24655 | 64734 | 17338 | 41705 | 77176 | 1365 | 17402 | 72655 | 39782 |
| ZR751 | 18986 | 2400 | 55498 | 10010 | 15010 | 4841 | 26278 | 85402 | 18598 | 22831 | 13121 | 853 | 14573 | 22615 | 28583 |
| T47D | 36520 | 2050 | 118007 | 39527 | 12071 | 4150 | 16084 | 69423 | 20773 | 15848 | 79611 | 354 | 11907 | 31549 | 29847 |
| MDAMB435S | 27417 | 4978 | 84390 | 32073 | 13132 | 46533 | 43056 | 106676 | 38135 | 32740 | 20664 | 709 | 20888 | 71378 | 37345 |
| DU4475 | 4305 | 7774 | 39818 | 11659 | 2880 | 30419 | 7945 | 44753 | 47627 | 16735 | 7491 | 1915 | 10075 | 14073 | 13893 |
| BT20 | 24453 | 1003 | 53910 | 12882 | 5640 | 4834 | 13954 | 59566 | 17156 | 9937 | 28290 | 758 | 10014 | 19635 | 40096 |
| BT474 | 10043 | 1151 | 44173 | 13646 | 6666 | 4360 | 15007 | 43592 | 21612 | 7568 | 17469 | 510 | 9343 | 19375 | 40387 |
| EVSAT | 23948 | 1495 | 68301 | 25554 | 26731 | 3212 | 27993 | 90825 | 48301 | 7636 | 3201 | 718 | 16233 | 31021 | 50256 |
| BT549 | 194081 | 2428 | 69592 | 23070 | 15953 | 6370 | 19238 | 113278 | 74131 | 21315 | 136091 | 1637 | 17477 | 40632 | 17143 |
| HCT116 | 6043 | 2432 | 61149 | 30911 | 17912 | 6212 | 26003 | 142163 | 42807 | 28728 | 33560 | 5623 | 25083 | 49463 | 68026 |
| HT29 | 10348 | 1468 | 61685 | 30958 | 16409 | 4686 | 17715 | 142199 | 20390 | 24951 | 70335 | 2365 | 23088 | 28163 | 40607 |
| SW480 | 10928 | 1460 | 89492 | 27923 | 5250 | 11118 | 12309 | 86735 | 34576 | 16381 | 17143 | 2054 | 16663 | 38854 | 30307 |
| CACO2 | 2789 | 1039 | 79792 | 56214 | 15305 | 5458 | 26333 | 126843 | 25147 | 27243 | 6094 | 788 | 19952 | 48037 | 66877 |
| MIAPACA2 | 26051 | 2026 | 90363 | 17241 | 19901 | 15437 | 20673 | 84369 | 48139 | 21334 | 220992 | 2593 | 10082 | 27472 | 22020 |
| DU145 | 37692 | 1264 | 74630 | 23958 | 16146 | 3283 | 29583 | 59734 | 23923 | 13762 | 34232 | 1090 | 17158 | 28659 | 20380 |
| PC3 | 44158 | 3135 | 138850 | 21906 | 8125 | 4682 | 16238 | 82311 | 23028 | 14902 | 16218 | 1699 | 13666 | 29622 | 20872 |
| HELA | 121220 | 1472 | 58582 | 13764 | 12594 | 5549 | 13497 | 98598 | 27530 | 32021 | 34040 | 1418 | 15962 | 33338 | 36371 |
| Caki2 | 38288 | 456 | 55322 | 5236 | 3638 | 1368 | 10154 | 82337 | 12493 | 7692 | 91219 | 1494 | 10381 | 18825 | 53870 |
| 7860 | 61859 | 2019 | 60653 | 21260 | 9191 | 4275 | 17497 | 126796 | 35884 | 26449 | 99063 | 1487 | 19024 | 31310 | 37510 |
| A375 | 23971 | 1896 | 21361 | 15167 | 5261 | 4876 | 9670 | 54050 | 14856 | 17504 | 30583 | 2379 | 10226 | 13748 | 15661 |
| NCIH226 | 12000 | 457 | 12715 | 4480 | 1936 | 3631 | 6463 | 29207 | 10843 | 5495 | 68710 | 3729 | 2373 | 7042 | 12122 |
| NCIH460 | 15460 | 861 | 52204 | 13749 | 16543 | 3770 | 12572 | 70461 | 18076 | 15136 | 37452 | 1791 | 10080 | 18243 | 18752 |
| NCIH810 | 26361 | 4148 | 28540 | 9799 | 6610 | 13164 | 13379 | 82085 | 17351 | 14805 | 24670 | 1170 | 6683 | 12546 | 17568 |
| NCIH1838 | 19457 | 2385 | 37599 | 11013 | 6007 | 10721 | 18382 | 38632 | 14779 | 7356 | 12274 | 929 | 5222 | 11945 | 14544 |
| NCIH2030 | 31745 | 1316 | 77476 | 17810 | 11256 | 4315 | 16730 | 63120 | 20314 | 14020 | 17601 | 768 | 9569 | 19200 | 14457 |
| NCIH358 | 2978 | 811 | 10373 | 4377 | 2687 | 2302 | 7778 | 28761 | 5790 | 2502 | 11214 | 2965 | 2668 | 3651 | 9326 |
| NCIH1793 | 29263 | 757 | 37528 | 4437 | 4627 | 2706 | 3998 | 39807 | 6678 | 6456 | 33713 | 564 | 3473 | 8382 | 14439 |
| NCIH2347 | 9414 | 2273 | 46740 | 11895 | 2861 | 3715 | 9649 | 51794 | 10778 | 10672 | 36595 | 2670 | 9197 | 13434 | 15359 |
| NCIH1975 | 11153 | 860 | 22119 | 9259 | 3351 | 4156 | 6866 | 28054 | 9920 | 7841 | 18737 | 1055 | 3644 | 7567 | 14079 |
| A549 | 15550 | 754 | 72577 | 18539 | 7978 | 3631 | 12320 | 87282 | 17876 | 27484 | 71674 | 2117 | 14901 | 28781 | 27668 |
| NCIH23 | 591 | 314 | 1730 | 2410 | 2863 | 2910 | 8499 | 6803 | 5496 | 684 | 23491 | 3220 | 560 | 652 | 13043 |
| NCIH2228 | 3683 | 454 | 20158 | 7261 | 2784 | 4287 | 10381 | 14942 | 12681 | 5687 | 33141 | 1758 | 2147 | 2899 | 15074 |
| NCIH661 | 14055 | 349 | 25401 | 7646 | 3631 | 6124 | 7484 | 51939 | 22198 | 9237 | 22777 | 847 | 5371 | 11217 | 12406 |
| NCIH1703 | 22165 | 1306 | 51213 | 14004 | 10886 | 6446 | 16155 | 58119 | 32471 | 19185 | 47070 | 1016 | 9976 | 27116 | 12854 |
| NCIH1581 | 27076 | 281 | 35495 | 14836 | 5182 | 3738 | 10878 | 57567 | 30903 | 11933 | 237 | 1092 | 4047 | 11377 | 17791 |
| NCIH1563 | 23670 | 731 | 19393 | 4992 | 3926 | 2293 | 7637 | 21475 | 4905 | 6563 | 56499 | 520 | 1627 | 3645 | 14726 |
| NCIH522 | 25713 | 770 | 14011 | 15357 | 4046 | 2734 | 16046 | 47912 | 26801 | 6326 | 2517 | 1574 | 5067 | 5606 | 15596 |
| NCIH1437 | 7590 | 1149 | 48497 | 19166 | 4238 | 2805 | 7674 | 52435 | 10532 | 20575 | 34322 | 1506 | 7952 | 7466 | 13929 |
| NCIH44! | 16038 | 1345 | 54481 | 11813 | 5527 | 3062 | 11044 | 54583 | 13096 | 10804 | 2698 | 1320 | 7427 | 11949 | 17031 |
| NCIH69 | 62350 | 4890 | 30152 | 11463 | 5383 | 1824 | 9499 | 57809 | 19871 | 11820 | 5823 | 493 | 4486 | 8939 | 22812 |
| NCIH1048 | 33156 | 3199 | 45362 | 6950 | 3987 | 2675 | 11361 | 59474 | 20308 | 12581 | 55503 | 710 | 8813 | 19372 | 27484 |
| NCIH2170 | 4690 | 572 | 31098 | 10264 | 5525 | 1885 | 8737 | 79720 | 6074 | 8374 | 1405 | 578 | 7187 | 9461 | 19383 |
| SKMES1 | 16719 | 1311 | 30074 | 11612 | 4508 | 7353 | 8146 | 54256 | 17518 | 14253 | 110152 | 2088 | 8000 | 18861 | 14543 |
| CHAGOK1 | 13277 | 1276 | 32460 | 11366 | 7571 | 2689 | 6736 | 63418 | 19372 | 13970 | 16762 | 881 | 6436 | 15806 | 13886 |

### Beispiel

**Tab. 5. Ergebnisse der Korrelationsanalysen**

| Gen | alle Zelllinien | alle Lungenzelllinien | Brustzelllinien |
|---|---|---|---|
| CCNE2 | keine Korrelation | keine Korrelation | r = 0.79 |
| | | | P = 0.0003 |
| | | | hoch signifikant |
| MAD2L2 | keine Korrelation | keine Korrelation | r = 0.74 |
| | | | P = 0.00097 |
| | | | hoch signifikant |

Die Sensitivität von 51 humanen Tumorzelllinien gegenüber der Verbindung A wurde in Proliferationsassays bestimmt. Die ermittelten IC50 Werte wurden mit den relativen mRNA Mengen von 62 Zellzyklus-regulatorischer Proteine, die in unabhängigen Genchip-Hybridisierungsstudien (Affymetrix-Technologie) bestimmt worden waren, korreliert. Gene, für die statistisch signifikante Korrelationen (P < 0.05) innerhalb der Brusttumorzelllinien gefunden wurden sind in der Tabelle 5 zusammengefasst. Die Korrelationskoeffizienten und Signifikanzen wurden mit Hilfe Microsoft Excel 2003 und SigmaStat 3.0 berechnet.

Über alle analysierten Zelllinien betrachtet, sowie in den Teilgruppen der Lungenzelllinien findet sich keine Korrelation zwischen der mRNA Menge der Gene CCNE2 (Cyclin E2) oder MAD2L2 und dem IC50 der Zelllinien gegenüber der Verbindung A. Überraschenderweise zeigt die Korrelationsanalyse für die Teilgruppe der 16 Brusttumorzellinien eine statistisch hoch signifikante Korrelation der mRNA Menge der Gene CCNE2 oder MAD2L2 mit der als IC50 bestimmten Sensitivität der Zellen gegenüber der Verbindung A (Tab. 5.).

Diese Daten belegen, dass die relativen mRNA Mengen der Gene CCNE2 und/oder MAD2L2 die Sensitivität von humanen Brusttumorzellen gegenüber der Verbindung A anzeigen können. Eine hohe relative mRNA Menge der Gene CCNE2 und/oder MAD2L2, für die ein positiver Korrelationskoeffizient gefunden wurde, zeigt einen höheren IC50 gleichbedeutend mit einer geringeren Sensitivität der Zellen gegenüber der Verbindung A an.

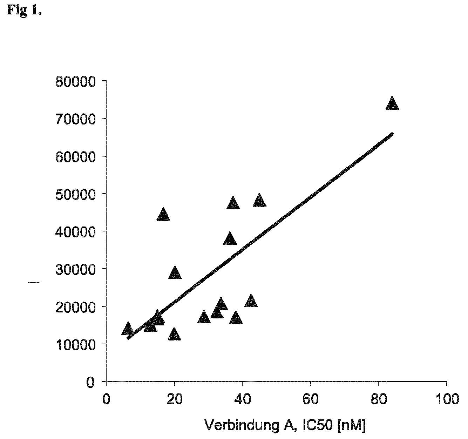

Figuren
- Fig. 1.: Grafische Darstellung der Sensitivität der humanen Brusttumorzellinien gegenüber der Verbindung A bestimmt als IC 50 [nM] in Proliferationsassays gegen die relative mRNA Menge des Gens MAD2L2 .Die durchgezogene Linie stellt die Korrelationsgerade dar.

## Patentansprüche

1. Verwendung von MAD2L2 als Stratifikationsmarker bei der Behandlung von Brusttumoren mit einer Verbindung der allgemeinen Formel (I) 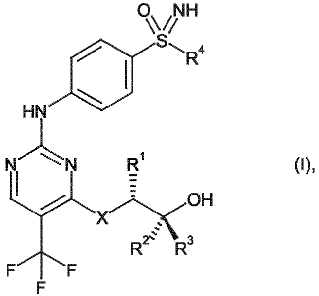 in der
X für -O- oder NH- steht, und
R¹ für eine Methyl-, Ethyl-, Propyl- oder Isopropylgruppe steht, und
R² und R³ unabhängig voneinander für Wasserstoff, eine Methyl- oder Ethylgruppe stehen, und
R⁴ für eine C₁-C₆-Alkylgruppe oder einen C₃-C₇-Cycloalkylring steht,
oder mit einem ihrer physiologisch verträglichen Salze, Diastereomere oder Enantiomere.

2. Verwendung gemäß Anspruch 1 bei der Behandlung mit einer Verbindung der allgemeinen Formel (I), in der
X für -O- oder NH- steht, und
R¹ für eine Methylgruppe steht, und
R² für eine Methylgruppe steht, und
R³ für Wasserstoff oder eine Methylgruppe steht, und
R⁴ eine Methyl- oder Ethylgruppe oder für einen Cyclopropyking steht,
oder mit einem ihrer physiologisch verträglichen Salze, Diastereomer oder Enantiomere.

3. Verwendung gemäß Anspruch 1 bei der Behandlung mit (2R,3R)-3-{[2-{[4-(R-Cyclopropylsulfonimidoyl)phenyl]amino}-5-(trifluormethyl)pyrimidin-4-yl]oxy}butan-2-ol.

4. Verwendung gemäß einem der Ansprüche 1 bis 3 bei der Behandlung von Brusttumoren in Mono- oder in Kombinationstherapie.

5. Verfahren zur Selektion von Bruststumor-Patienten, die auf eine Behandlung mit einer Verbindung der Formel (I) ansprechen könnten, **dadurch gekennzeichnet** dasss die Expressionshöhe von MAD2L2 bestimmt wird.

6. Verfahren nach Anspruch 5, **dadurch gekennzeichnet** dasss die Expressionshöhe von MAD2L2 auf Nukleinsäureebene bestimmt wird.

7. Verfahren nach Anspruch 5, **dadurch gekennzeichnet** dasss die Expressionshöhe von MAD2L2 auf Proteinebene bestimmt wird.

8. Verfahren nach Anspruch 5, **dadurch gekennzeichnet** dasss die Expressionshöhe von MAD2L2 an einer Probe aus der Zellkultur bestimmt wird.

9. Verfahren nach Anspruch 5, **dadurch gekennzeichnet** dasss die Expressionshöhe von MAD2L2 an einer Probe aus einem Säuger-Organismus bestimmt wird.

10. Verfahren nach Anspruch 5, **dadurch gekennzeichnet** dasss die Expressionshöhe von MAD2L2 an einer Probe aus einem menschlichen Patienten bestimmt wird.

11. Verfahren nach Anspruch 5, **dadurch gekennzeichnet** dasss die Expressionshöhe von MAD2L2 an einer Probe aus einer Kultur von Zellen oder aus einem Versuchstier bestimmt wird.

## Claims

1. Use of MAD2L2 as stratification marker in the treatment of breast tumours with a compound of the general formula (I) 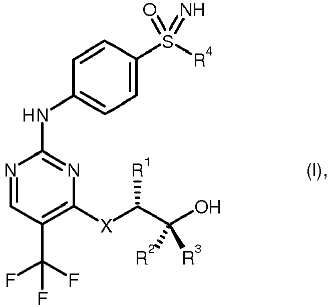 in which
X represents -O- or -NH-, and
R¹ represents a methyl, ethyl, propyl or isopropyl group, and
R² and R³ independently of one another represent hydrogen, a methyl or ethyl group, and
R⁴ represents a C₁-C₆-alkyl group or a C₃-C₇-cycloalkyl ring,
or with one of its physiologically acceptable salts, diastereomers or enantiomers.

2. Use according to Claim 1 in the treatment with a compound of the general formula (I) in which
X represents -O- or -NH-, and
R¹ represents a methyl group, and
R² represents a methyl group, and
R³ represents hydrogen or a methyl group, and
R⁴ represents a methyl or ethyl group or represents a cyclopropyl ring,
or with one of its physiologically acceptable salts, diastereomers or enantiomers.

3. Use according to Claim 1 in the treatment with (2R,3R)-3-{[2-{[4-(R-cyclopropylsulphonimidoyl)phenyl]amino}-5-(trifluoromethyl)pyrimidin-4-yl]oxy}butan-2-ol.

4. Use according to any of Claims 1 to 3 in the treatment of breast tumours in monotherapy or in combination therapy.

5. Method for the selection of breast tumour patients who may respond to treatment with a compound of the formula (I), **characterized in that** the extent of expression of MAD2L2 is determined.

6. Method according to Claim 5, **characterized in that** the extent of expression of MAD2L2 is determined at the nucleic acid level.

7. Method according to Claim 5, **characterized in that** the extent of expression of MAD2L2 is determined at the protein level.

8. Method according to Claim 5, **characterized in that** the extent of expression of MAD2L2 is determined on a sample from the cell culture.

9. Method according to Claim 5, **characterized in that** the extent of expression of MAD2L2 is determined on a sample from a mammalian organism.

10. Method according to Claim 5, **characterized in that** the extent of expression of MAD2L2 is determined on a sample from a human patient.

11. Method according to Claim 5, **characterized in that** the extent of expression of MAD2L2 is determined on a sample from a culture of cells or from an experimental animal.

## Revendications

1. Utilisation de MAD2L2 en tant que marqueur de stratification lors du traitement de tumeurs du sein avec un composé de formule générale (I) 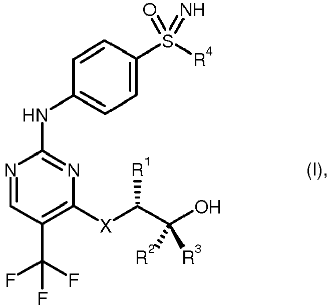 dans laquelle
X représente -O- ou -NH-, et
R¹ représente un groupe méthyle, éthyle, propyle ou isopropyle, et
R² et R³ représentent chacun indépendamment de l'autre un atome d'hydrogène, un groupe méthyle ou éthyle, et
R⁴ représente un groupe alkyle en C₁-C₆ ou un noyau cycloalkyle en C₃-C₇,
ou avec l'un de ses sels, diastéréoisomères ou énantiomères physiologiquement compatibles.

2. Utilisation selon la revendication 1 pour le traitement avec un composé de formule générale (I), dans laquelle
X représente -O- ou -NH-, et
R¹ représente un groupe méthyle, et
R² représente un groupe méthyle, et
R³ représente un atome d'hydrogène ou un groupe méthyle, et
R⁴ représente un groupe méthyle ou éthyle ou un noyau cyclopropyle,
ou avec l'un de ses sels, diastéréoisomères ou énantiomères physiologiquement compatibles.

3. Utilisation selon la revendication 1 lors du traitement avec le (2R, 3R)-3-{[2-{[4-(R-cyclopropylsulfonimidoyl)phényl]amino}-5-(trifluorométhyl)pyrimidin-4-yl]oxy}butan-2-ol.

4. Utilisation selon l'une des revendications 1 à 3 lors du traitement de tumeurs du sein dans le cadre d'une monothérapie ou d'une thérapie en association.

5. Procédé de sélection de patients présentant une tumeur du sein, qui pourraient répondre à un traitement avec un composé de formule (I), **caractérisé en ce qu'**on détermine le niveau d'expression de MAD2L2.

6. Procédé selon la revendication 5, **caractérisé en ce qu'**on détermine le niveau d'expression de MAD2L2 au plan des acides nucléiques.

7. Procédé selon la revendication 5, **caractérisé en ce qu'**on détermine le niveau d'expression de MAD2L2 au plan des protéines.

8. Procédé selon la revendication 5, **caractérisé en ce qu'**on détermine le niveau d'expression de MAD2L2 sur un échantillon provenant de la culture cellulaire.

9. Procédé selon la revendication 5, **caractérisé en ce qu'**on détermine le niveau d'expression de MAD2L2 sur un échantillon provenant d'un organisme mammifère.

10. Procédé selon la revendication 5, **caractérisé en ce qu'**on détermine le niveau d'expression de MAD2L2 sur un échantillon provenant d'un patient humain.

11. Procédé selon la revendication 5, **caractérisé en ce qu'**on détermine le niveau d'expression de MAD2L2 sur un échantillon provenant d'une culture de cellules ou d'un animal d'expérience.
